# EUROPÄISCHE PATENTSCHRIFT

(11) **EP 1 911 493 B1**
(45) Veröffentlichungstag und Bekanntmachung des Hinweises auf die Patenterteilung: **28.10.2009**
(21) Anmeldenummer: 07116397.6
(22) Anmeldetag: 14.09.2007
(51) Int. Cl.: A61N 5/10

(54) **Verfahren zur Bestimmung der Reichweite von Strahlung**
Method for determining the range of radiation
Procédé destiné à la détermination du rayon d'action du rayonnement

(30) Priorität: 12.10.2006 DE 102006048426
(43) Veröffentlichungstag der Anmeldung: 16.04.2008
(73) Patentinhaber: SIEMENS AKTIENGESELLSCHAFT, 80333 München (DE)
(72) Erfinder: Rietzel, Eike Dr., 64289 Darmstadt (DE)

(56) Entgegenhaltungen:
- US-A- 5 673 303
- US-A- 5 727 041
- US-A1- 2005 192 764
- US-A1- 2007 049 839
- US-B1- 6 215 841
- JÄKEL O ET AL: "Relation between carbon ion ranges and x-ray CT numbers" MEDICAL PHYSICS, AIP, MELVILLE, NY, US, Bd. 28, Nr. 4, April 2001 (2001-04), Seiten 701-703, XP012011450 ISSN: 0094-2405

## Beschreibung

Die Erfindung betrifft ein Verfahren zur Bestimmung der Reichweite von Strahlung, insbesondere in der Partikeltherapie, sowie eine zur Durchführung des Verfahrens geeignete Vorrichtung.

Mit der Reichweite von Partikelstrahlung in verschiedenen Phantom-Materialien sowie in Knochen befasst sich beispielsweise der Artikel "Relation between carbon ion ranges and x-ray CT numbers" (O. Jäkel, C. Jacob, D. Schardt, C. P. Karger, G. H. Hartmann; Medical Physics, Vol. 28, No. 4, April 2001, S. 701 - 703). Mit den Phantom-Materialien wurden verschiedene Arten von Gewebe, unter anderem Muskeln, Fett und Lunge, simuliert. Reichweitenmessungen wurden mit Hilfe eines Absorbers, welcher Wasser in einer variablen Schichtdicke enthält und zusätzlich zu dem Phantom-Material zwischen zwei Ionisationskammern positioniert wurde, durchgeführt, wobei die Bestrahlung senkrecht zu den zur Ladungsmessung verwendeten Ionisationskammern erfolgte. Damit wurde eine Relation zwischen der computertomographisch bestimmbaren Schwächung von Röntgenstrahlung und der Reichweite von Partikeln, nämlich Kohlenstoffionen und Protonen, in verschiedenen Materialien ermittelt.

Aus der DE 10 2004 057 726 A1 ist eine medizinische Untersuchungs- und Behandlungseinrichtung bekannt, welche eine Partikelstrahlung, insbesondere Ionenstrahlung, emittierende Strahlungsquelle umfasst. Zusätzlich weist die Untersuchungs- und Behandlungseinrichtung einen auf der der Strahlungsquelle gegenüberliegenden Seite des Zielvolumens angeordneten Röntgenstrahler sowie einen Detektor auf. Dies hat den Zweck, sowohl eine zeitsparende Durchleuchtung als auch eine präzise Strahlentherapie zu ermöglichen. Während eine Überwachung des Bestrahlungsprofils des Partikelstrahls vorgesehen ist, ist die Bestimmung der Reichweite der Partikelstrahlung jedoch nicht Gegenstand der DE 10 2004 057 726 A1.

In der Partikelstrahlentherapie ist es von besonderer Bedeutung, die Reichweite der verwendeten Strahlung im Zielvolumen möglichst genau zu prognostizieren. Im Unterschied zu elektromagnetischer Strahlung weist Partikelstrahlung ein sogenanntes invertiertes Dosisprofil auf: Die im der Strahlung ausgesetzten Zielvolumen deponierte Strahlendosis wächst mit zunehmender Eindringtiefe und erreicht kurz vor der maximalen Reichweite ein scharfes Maximum. Dies hat einerseits den Vorteil, dass im Strahlengang vor dem zu behandelnden Zielvolumen angeordnetes Gewebe besonders geschont wird. Andererseits erfordert eine optimale Bestrahlungsplanung eine genaue Kenntnis der Lage des Maximums des Dosisprofils.

Der Erfindung liegt die Aufgabe zugrunde, die Reichweite von Strahlung, insbesondere Partikelstrahlung, in einem hinsichtlich der strahlungsbeeinflussenden Eigenschaften inhomogenen Zielvolumen besonders genau zu bestimmen.

Diese Aufgabe wird erfindungsgemäß gelöst durch ein Verfahren mit den Merkmalen des Anspruchs 1 sowie durch eine zur Durchführung dieses Verfahrens geeignete Vorrichtung mit den Merkmalen des Anspruchs 9.

Das Verfahren, mit welchem die Reichweite von Strahlung, insbesondere Partikelstrahlung, bestimmt wird, geht von der Gliederung eines zu bestrahlenden Zielvolumens in eine Mehrzahl von Volumenelementen, auch als Voxel bezeichnet, aus. Die einzelnen Voxel können beispielsweise quaderförmig sein. Ebenso ist jedoch auch die Definition nicht quaderförmiger Voxel von nicht notwendigerweise einheitlicher Größe möglich.

Die Einteilung in Volumenelemente wird zunächst verwendet, um eine ortsaufgelöste Information über strahlungsschwächende Eigenschaften des Zielvolumens zu erhalten. Um diese Information zu erhalten, wird das Zielvolumen jedoch nicht der Strahlung, deren Reichweite zu bestimmen ist, ausgesetzt, sondern auf andere Weise, beispielsweise röntgentechnisch, insbesondere durch Computertomographie, untersucht. Die strahlungsschwächenden Eigenschaften eines jeden untersuchten Voxels werden bevorzugt in an sich bekannten Hounsfield-Einheiten klassifiziert (s. beispielsweise: "Medizinische Informatik, Biometrie und Epidemologie"; hrsg. von Hans-Jürgen Seelos, Walter de Gruyter & Co., Berlin, 1997, S. 71). Der Wertebereich der Hounsfield-Einheiten (HU) reicht von minus 1000 (äußerst geringe Schwächung von Röntgenstrahlung) bis beispielsweise plus 2000 (sehr starke Schwächung von Röntgenstrahlung), wobei der Wert von Wasser definitionsgemäß auf Null festgelegt ist.

Weiter wird im Rahmen des Verfahrens aus den spezifisch für die einzelnen Voxel ermittelten strahlungsschwächenden Eigenschaften eine Reichweiteninformation hergeleitet, welche sich auf die Reichweite derjenigen Strahlung, insbesondere Partikelstrahlung, bezieht, mit der das Zielvolumen zu einem späteren Zeitpunkt zu behandeln ist. Die Reichweiteninformation wird bevorzugt als wasseräquivalente Reichweite (WEPL, water equivalent path length) bestimmt. Die Zuordnung von Reichweiteninformation zu strahlungsschwächenden Eigenschaften stützt sich beispielsweise auf zuvor experimentell und/oder theoretisch gewonnene Daten. Diesbezüglich wird auf den eingangs zitierten Artikel von Jäkel et al. hingewiesen. Vorzugsweise wird die Relation zwischen in Hounsfield-Einheiten ausgedrückten strahlungsschwächenden Eigenschaften und wasseräquivalenten Reichweiten, bezogen auf eine bestimmte Strahlung, in Tabellenform als sogenannte Kalibrierkurve bereitgehalten.

Ist die für einen ersten Voxel gewonnene Reichweiteninformation mit den Reichweiteninformationen der umgebenden Voxel identisch, so wird die Reichweiteninformation des ersten Voxels nicht geändert. Unterscheidet sich die Reichweiteninformation des ersten Voxels jedoch von den Reichweiteninformationen zweier dem ersten Voxel benachbarter Voxel in unterschiedlicher Weise, so wird die Reichweiteninformation des ersten Voxels geändert. Die geänderten Zuordnungen zwischen strahlungsschwächenden Eigenschaften und Reichweiteninformation bilden insgesamt eine zweite Kalibrierkurve. Diese kann entweder wie die erste Kalibrierkurve vollständig gespeichert sein oder bedarfsweise für einzelne Werte aus der ersten Kalibrierkurve generiert werden.

Insbesondere wird die dem ersten Voxel zugeordnete wasseräquivalente Reichweite korrigiert, wenn für einen ersten benachbarten Voxel eine geringere wasseräquivalente Reichweite und für einen zweiten benachbarten Voxel eine im Vergleich zum ersten Voxel höhere wasseräquivalente Reichweite bestimmt wurden, wobei die benachbarten Voxel auf sich gegenüberliegenden Seiten des ersten Voxels angeordnet sind.

Im letztgenannten Fall, in welchem die für einen Voxel bestimmte wasseräquivalente Reichweite zwischen Werten liegt, die für diesem Voxel benachbarte Voxel ermittelt wurden, ist mit erheblicher Wahrscheinlichkeit ein sogenannter Partialvolumeneffekt gegeben. Dies gilt insbesondere dann, wenn einem der benachbarten Voxel ein sehr geringer Wert auf der Hounsfield-Skala und dem weiteren benachbarten Voxel ein sehr hoher Wert auf dieser Skala zugeordnet wurde. Der geringe Wert auf der Hounsfield-Skala entspricht dabei einem geringen Wert der wasseräquivalenten Reichweite, während sich der sehr hohe Wert auf der Hounsfield-Skala in einem hohen Wert der wasseräquivalenten Reichweite ausdrückt. Der Partialvolumeneffekt bedeutet, dass dem mittleren Voxel Stoffeigenschaften zugeordnet werden, die zwischen den Eigenschaften der diesem Voxel benachbarten, einander gegenüberliegenden Voxel liegen.

Tatsächlich jedoch befindet sich in dem Voxel, in welchem der Partialvolumeneffekt auftritt, kein dritter Stoff, dessen Eigenschaften zwischen den Eigenschaften der Stoffe liegen, die die benachbarten Voxel ausfüllen, sondern lediglich eine Grenzfläche zwischen den in den benachbarten Voxeln detektierten Stoffen. Würden die benachbarten Voxel nicht in die Auswertung eingehen, so wäre eine Fehlinterpretation der dem mittleren Voxel zugeordneten strahlungsschwächenden Eigenschaften die Folge. Als Auswirkung dieser Fehlinterpretation würde einem Voxel, durch welchen eine Grenzfläche verläuft, eine unzutreffende, in der Regel zu hohe wasseräquivalente Reichweite zugeordnet werden.

Um eine derartige Fehlinterpretation zu vermeiden, wird nach dem erfindungsgemäßen Verfahren bei der auch als Kalibrierung bezeichneten Zuordnung zwischen pro Voxel detektierten strahlungsschwächenden Eigenschaften und wasseräquivalenten Reichweiten auch die Umgebung des zu kalibrierenden Voxels berücksichtigt. Die Einbeziehung von Daten umgebender Voxel bei der Kalibrierung der Hounsfield Einheiten in Wasseräquivalenz ist unter Nutzung von Algorithmen realisierbar, die prinzipiell in der Bildverarbeitung verwendet werden. Beispielhaft ist die adaptive Filterung zu nennen, welche zur Festlegung des Filterergebnisses die Umgebung innerhalb einer bestimmten Maske verwendet.

Dadurch, dass bei der zur Vorbereitung der Bestrahlung notwendigen Kalibrierung die für einen bestimmten Voxel verwendeten Kalibrierdaten nicht ausschließlich von für diesen Voxel bestimmten Messdaten, sondern auch von Messdaten, insbesondere Computertomographie-Daten, benachbarter Voxel abhängig sind, werden somit besonders Grenzflächeneffekte berücksichtigt. Damit werden Unter- und Überreichweiten, welche zu Fehldosierungen, das heißt Unterdosierungen im Zielvolumen und Überdosierungen im umgebenden Gewebe, führen würden, vermieden. Die spezifisch für einzelne Voxel, abhängig von deren jeweiliger Umgebung, vorgenommene Korrektur der Kalibrierdaten kann im Einzelfall dazu führen, dass sich die Partikelreichweite um einen Betrag ändert, welcher größer als die in Strahlungsrichtung gemessene Ausdehnung eines Voxels ist. Als Vergleichssituation wird hierbei von einer Bestrahlung ausgegangen, die auf einer Kalibrierung basiert, die zwar die für einzelne Voxel detektierten unterschiedlichen Stoffeigenschaften berücksichtigt, jedoch keine umgebungsabhängigen Korrekturen vornimmt.

Der Vorteil der Erfindung liegt insbesondere darin, dass eine hohe Präzision in der Partikeltherapie erreicht wird, indem zur Zuordnung ortsaufgelöst gemessener strahlungsschwächender Eigenschaften zu prognostizierten wasseräquivalenten Reichweiten mehrere Kalibrierkurven zur Verfügung gestellt werden, wobei eine automatische Auswahl der geeigneten Kalibrierkurve anhand eines Vergleiches der gemessenen strahlungsschwächenden Eigenschaften eines Volumenelementes mit den strahlungsschwächenden Eigenschaften umgebender Volumenelemente getroffen wird.

Nachfolgend wird ein Ausführungsbeispiel der Erfindung anhand einer Zeichnung näher erläutert. Hierin zeigen:
- FIG 1: eine zur Durchführung des erfindungsgemäßen Verfahrens ausgebildete medizinische Untersuchungs- und Behandlungseinrichtung,
- FIG 2: einen Schnitt durch das mittels der Untersuchungs- und Behandlungseinrichtung nach FIG 1 behandelte Zielvolumen, und
- FIG 3: in einem Diagramm den Zusammenhang zwischen durch Messung ermittelten strahlungsschwächenden Eigenschaften des Zielvolumens und hieraus hergeleiteten Reichweiteninformationen.

Einander entsprechende Teile oder Parameter sind in allen Figuren mit den gleichen Bezugszeichen gekennzeichnet.

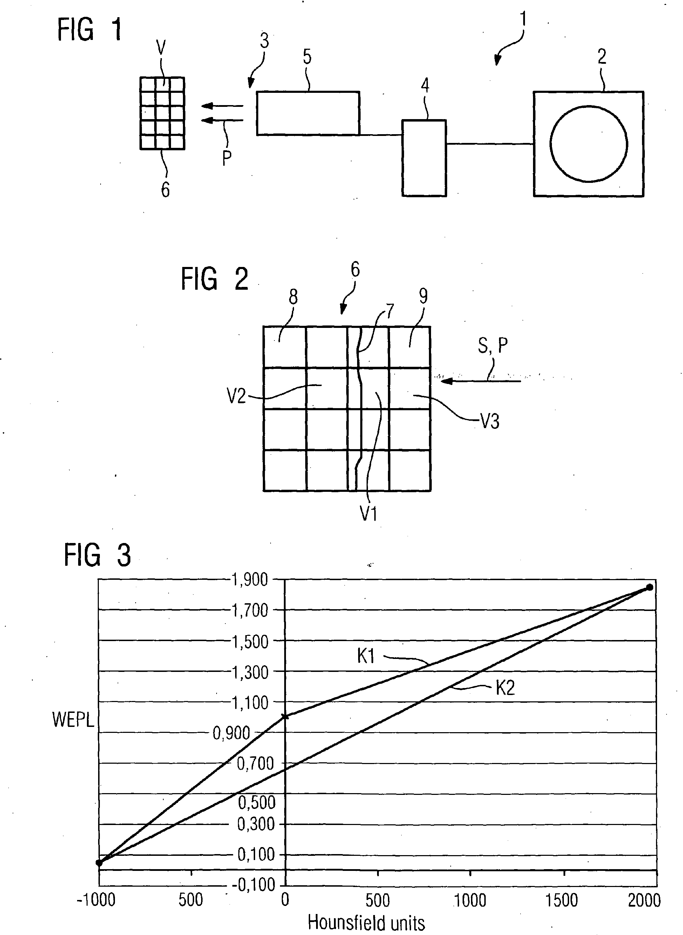

Die FIG 1 zeigt symbolisiert eine medizinische Untersuchungs- und Behandlungseinrichtung 1, welche sich im Wesentlichen aus einer Diagnoseeinrichtung 2, nämlich einem Computertomographen, einer Bestrahlungsanlage als Therapieeinrichtung 3, sowie einer die Diagnoseeinrichtung 2 mit der Therapieeinrichtung 3 verknüpfenden Recheneinheit 4 zusammensetzt. Abweichend von der symbolisierten Darstellung kann es sich bei der Recheneinheit 4 auch um ein größeres Datenverarbeitungsnetzwerk, beispielsweise integriert in ein Radiologieinformationssystem und/oder Krankenhausinformationssystem, handeln. Die Bestrahlungsanlage 3 umfasst eine Strahlenquelle 5, welche Partikelstrahlung P, beispielsweise Karbonionenstrahlung, emittiert, die auf ein zu behandelndes Zielvolumen 6 trifft. Mittels eines kartesischen Koordinatensystems ist das Zielvolumen 6 in eine Vielzahl quaderförmiger Voxel V unterteilt. Die Reichweite der Partikelstrahlung P im Zielvolumen 6 wird mit Hilfe der Recheneinheit 4 bestimmt, welche programmtechnisch zur Durchführung des im Folgenden erläuterten Verfahrens eingerichtet ist.

Der Patient, der mit der Therapieeinrichtung 3 zu bestrahlen ist, wird zunächst mittels der Diagnoseeinrichtung 2, welche zur Durchführung eines bildgebenden medizintechnischen Verfahrens ausgebildet ist, untersucht. Hierbei wird ein Volumendatensatz aufgenommen, wobei innerhalb eines bestimmten Volumens, nämlich des bei der späteren Bestrahlung als Zielvolumen 6 bezeichneten Volumens, jedem Voxel V ein Grauwert zugeordnet wird, der - ausgedrückt in Hounsfield Einheiten (HU, Hounsfield units) - die Schwächung der Röntgenstrahlung in dem betreffenden Voxel V angibt und damit eine Dichteinformation liefert.

Aus der in Hounsfield Einheiten angegebenen Schwächung der Röntgenstrahlung wird unter Nutzung empirischer Daten, die beispielsweise in sogenannte "Hounsfield look-up tables" Eingang gefunden haben, zunächst separat für jeden Voxel V ein Wert ermittelt, der die wasseräquivalente Reichweite (WEPL) der Partikelstrahlung P angibt. Wäre im einfachsten Fall das zu bestrahlende Gewebe, das heißt das Zielvolumen 6, vollkommen homogen, so könnte die Reichweite der Partikelstrahlung P direkt aus dem Wert der wasseräquivalenten Reichweite bestimmt werden.

Tatsächlich sind jedoch praktisch in jedem Fall Inhomogenitäten des Zielvolumens 6 vorhanden. Eine solche komplexere Situation einer Bestrahlung des Zielvolumens 6 mit der Partikelstrahlung P, von welcher ein einzelner Strahl S angedeutet ist, ist in FIG 2 skizziert. Innerhalb des Zielvolumens 6 ist eine Oberfläche 7 erkennbar, welche die Grenzfläche zwischen einem ersten Teilvolumen 8, in FIG 2 links der Oberfläche 7, und einem zweiten Teilvolumen 9 bildet. Beim ersten Teilvolumen 8 handele es sich um Knochen, beim zweiten Teilvolumen 9 um Luft. Der Strahl S durchläuft sowohl Luft als auch Knochen, wobei derjenige Voxel V, in welchem der Strahl S auf die Oberfläche 7 des Knochens trifft, mit V1 bezeichnet ist. An den ersten Voxel V1 grenzen auf einander gegenüberliegenden Seiten - in FIG 2 links beziehungsweise rechts des Voxels V1 - ein zweiter Voxel V2, vollständig innerhalb des Knochen, sowie ein dritter Voxel V3, vollständig außerhalb des Knochens. Alle Voxel V1,V2,V3 sind im Ausführungsbeispiel kubisch mit einer Kantenlänge von 1 mm. Dem zweiten Voxel V2 wird beispielsweise ein Hounsfield Wert von plus 1000 zugeordnet, während dem dritten, vollständig in der Luft befindlichen Voxel V3 ein Hounsfield Wert von minus 1000 zugeordnet wird. Der mittels Computertomographie bestimmte Hounsfield Wert des mittleren Voxels V1, durch welchen die Grenzfläche 7 zwischen Knochen und Luft verläuft, beträgt Null.

Um jedem Hounsfield Wert eine wasseräquivalente Reichweite (WEPL) zuzuordnen, kann auf eine in der Recheneinheit 4 gespeicherte, in FIG 3 dargestellte erste Kalibrierkurve K1 zugegriffen werden. Diese Kurve K1 stellt einen an sich bekannten Zusammenhang zwischen der Eigenschaft eines Materials, Röntgenstrahlung zu schwächen, und den Werten der wasseräquivalenten Reichweite dar und stimmt mit einer in der oben zitierten Veröffentlichung von Jäkel et al. (S. 702, FIG 2) wiedergegebenen Kurve überein. Nach der Kalibrierkurve K1 ist dem Hounsfield Wert Null ein Wert der wasseräquivalenten Reichweite von Eins zuzuordnen.

Der mit dem HU-Wert Null korrespondierende WEPL-Wert Eins ist definitionsgemäß zutreffend, wenn es sich bei dem betrachteten Material um Wasser handelt und mag auch für manche Arten von Weichteilgewebe zutreffend sein. Im vorliegenden Fall, in welchem sich im Voxel V1 weder Wasser noch Weichteilgewebe befindet, würde die Annahme des WEPL-Wertes Eins für den Voxel V1 jedoch zu einer die tatsächliche Zusammensetzung des Zielvolumens 6 nur unzureichend berücksichtigenden Bestrahlungsplanung führen.

Unter den gegebenen Umgebungsbedingungen, das heißt mit einem zentralen Voxel V1, der sowohl an einen Voxel V3 mit einem sehr viel niedrigeren Hounsfield Wert als auch an einen weiteren Voxel V2 mit sehr viel höherem Hounsfield Wert grenzt, ist abweichend von der ersten Kalibrierkurve K1 ein WEPL-Wert von 0,7, also ein um 30 % geringerer WEPL-Wert, zutreffend. Dieser korrigierte WEPL-Wert ist Teil einer zweiten Kalibrierkurve K2, die automatisch dann gewählt wird, wenn der Voxel V1 als auf einer Grenzfläche, hier der Oberfläche 7, liegend identifiziert wird. Als Bedingung für die Identifikation einer Grenzfläche ist vorzugsweise eine - insbesondere in Hounsfield Einheiten ausdrückbare - minimale Differenz zwischen den strahlungsschwächenden Eigenschaften des zentralen Voxels V1 und der diesem auf entgegengesetzten Seiten benachbarten Voxel V2,V3 festlegbar, beispielsweise softwaretechnisch einstellbar.

Wie aus FIG 3 hervorgeht, ist die Abweichung zwischen den umgebungsabhängig auszuwählenden Kalibrierkurven K1,K2 beim Hounsfield Wert Null am größten, während die Kalibrierkurven K1,K2 in Richtung zu weit im negativen und weit im positiven Bereich liegenden Hounsfield Werten hin konvergieren. Dies ist anschaulich damit zu erklären, dass in diesen Bereichen keine Grenzflächen zwischen Materialien mit extrem unterschiedlichen strahlungsschwächende Eigenschaften oder weit voneinander abweichenden WEPL-Werten existieren.

## Patentansprüche

1. Verfahren zur Bestimmung der Reichweite von Strahlung (P), mit folgenden Merkmalen:
- Ein zu bestrahlendes Zielvolumen (6) wird in eine Mehrzahl von Voxeln (V,V1,V2,V3) gegliedert,
- es werden, ohne das Zielvolumen (6) der Strahlung (P) auszusetzen, strahlungsschwächende Eigenschaften (HU), die einzelnen Voxeln (V,V1,V2,V3) zuzuordnen sind, bestimmt,
- aus den strahlungsschwächenden Eigenschaften (HU) wird eine Reichweiteninformation (WEPL) hergeleitet,
- unterscheidet sich die Reichweiteninformation (WEPL) eines ersten Voxels (V1) von der Reichweiteninformation (WEPL) zweier benachbarter Voxel (V2,V3) in unterschiedlicher Weise, so wird die Reichweiteninformation (WEPL) des ersten Voxels (V1) geändert.

2. Verfahren nach Anspruch 1,
**dadurch gekennzeichnet, dass** die Bestimmung der strahlungsschwächenden Eigenschaften des Zielvolumens (6) röntgentechnisch erfolgt.

3. Verfahren nach Anspruch 2,
**dadurch gekennzeichnet, dass** die Bestimmung der strahlungsschwächenden Eigenschaften mittels Computertomographie erfolgt.

4. Verfahren nach einem der Ansprüche 1 bis 3,
**dadurch gekennzeichnet, dass** die strählungsschwächenden Eigenschaften (HU) in Hounsfield Einheiten bestimmt werden.

5. Verfahren nach einem der Ansprüche 1 bis 4,
**dadurch gekennzeichnet, dass** die einem Voxel (V,V1,V2,V3) zugeordnete Reichweiteninformation (WEPL) als wasseräquivalente Reichweite bestimmt wird.

6. Verfahren nach Anspruch 5,
**dadurch gekennzeichnet, dass** eine Korrektur der Reichweiteninformation (WEPL) des ersten Voxels (V1) dann erfolgt, wenn für einen benachbarten Voxel (V3) eine geringere wasseräquivalente Reichweite (WEPL) und für einen weiteren benachbarten Voxel (V2) eine im Vergleich zum ersten Voxel (V1) höhere wasseräquivalente Reichweite (WEPL) bestimmt wurden.

7. Verfahren nach Anspruch 6,
**dadurch gekennzeichnet, dass** die dem ersten Voxel (V1) benachbarten, eine geringere beziehungsweise höhere wasseräquivalente Reichweite (WEPL) aufweisenden Voxel (V2,V3) auf entgegengesetzten Seiten des ersten Voxels (V1) angeordnet sind.

8. Verfahren nach Anspruch 7,
**dadurch gekennzeichnet, dass** als Korrektur eine Absenkung der dem ersten Voxel (V1) zugeordneten wasseräquivalenten Reichweite (WEPL) erfolgt.

9. Vorrichtung zur Bestimmung der Reichweite von Strahlung, insbesondere Partikelstrahlung, mit einer Recheneinheit (4), welche programmtechnisch zur Durchführung des Verfahrens nach Anspruch 1 eingerichtet ist.

## Claims

1. Method for determining the range of radiation (P) with the following features:
- a target volume (6) to be irradiated is subdivided into a plurality of voxels (V,V1,V2,V3),
- without exposing the target volume (6) to radiation (P), radiation attenuating characteristics (HU) are determined which are to be assigned to the individual voxels (V,V1,V2,V3),
- range information (WEPL) is derived from the radiation attenuating characteristics (HU),
- if the range information (WEPL) of a first voxel (V1) differs from the range information (WEPL) of two adjacent voxels (V2,V3) in different ways, the range information (WEPL) of the first voxel (V1) is changed.

2. Method according to claim 1,
**characterised in that** determination of the radiation attenuating characteristics of the target volume (6) takes place by means of X-ray technology.

3. Method according to claim 2,
**characterised in that** the determination of the radiation attenuating characteristics takes place by means of computed tomography.

4. Method according to one of claims 1 to 3,
**characterised in that** the radiation attenuating characteristics (HU) are determined in Hounsfield units.

5. Method according to one of claims 1 to 4,
**characterised in that** the range information (WEPL) assigned to a voxel (V,V1,V2,V3) is determined as a water equivalent range.

6. Method according to claim 5,
**characterised in that** a correction of the range information (WEPL) of the first voxel (V1) then takes place when a smaller water equivalent range (WEPL) is determined for a neighboring voxel (V3) and a larger water equivalent range (WEPL) is determined for a further neighboring voxel (V2) compared to the first voxel (V1).

7. Method according to claim 6,
**characterised in that** the voxels (V2, V3) next to the first voxel (V1) having a smaller or larger water equivalent range (WEPL) are arranged on opposite sides of the first voxel (V1).

8. Method according to claim 7,
**characterised in that,** as a correction, a reduction of the water equivalent range (WEPL) assigned to the first voxel (V1) takes place.

9. Device for determining the range of radiation, in particular of particle radiation, with a calculation unit (4) which is set up by programming to carry out the method according to claim 1.

## Revendications

1. Procédé pour déterminer la portée d'un rayonnement (P), ayant les caractéristiques suivantes:
- un volume cible (6) à irradier est divisé en une pluralité de voxels (V, V1, V2, V3),
- sans exposer le volume cible (6) au rayonnement (P), on détermine des propriétés d'atténuation de rayonnement (HU) qui sont associées à des oxels individuels (V, V1, V2, V3),
- on déduit une information sur la portée (WEPL) à partir des propriétés d'atténuation de rayonnement (HU)
- si l'information sur la portée (WEPL) d'un premier voxel (V1) diffère de façon différente, de l'information sur la portée (WEPL) de deuxièmes voxels voisins (V2, V3) alors l'information sur la portée (WEPL) du premier voxel (V1) est modifiée.

2. Procédé selon la revendication 1,
**caractérisé en ce que** la détermination des propriétés d'atténuation de rayonnement du volume cible se fait par une technique de rayons X.

3. Procédé selon la revendication 2,
**caractérisé en ce que** la détermination des propriétés d'atténuation de rayonnement du volume cible se fait au moyen d'une tomographie assistée par ordinateur.

4. Procédé selon l'une quelconque des revendications 1 à 3,
**caractérisé en ce que** les propriétés d'atténuation de rayonnement (HU) sont déterminées en unités Hounsfield.

5. Procédé selon l'une quelconque des revendications 1 à 4,
**caractérisé en ce que** l'information sur la portée (WEPL) associée à un voxel (V, V1, V2, V3) est déterminée sous la forme d'une portée équivalente dans l'eau.

6. Procédé selon la revendication 5,
**caractérisé en ce qu'**une correction de l'information sur la portée (WEPL) du premier voxel (V1) a alors lieu si on a déterminé une portée équivalente dans l'eau (WEPL) plus faible pour un voxel voisin (V3) et une portée équivalente dans l'eau (WEPL) plus élevée pour un autre voxel voisin (V2), par rapport à celle pour le premier voxel (V1).

7. Procédé selon la revendication 6,
**caractérisé en ce que** les voxels (V2, V3) voisins du premier voxel (V1), présentant une portée équivalente dans l'eau (WEPL) plus faible, respectivement plus élevée sont disposés sur les côtés opposés du premier voxel (V1).

8. Procédé selon la revendication 7,
**caractérisé en ce qu'**une diminution de la portée équivalente dans l'eau (WEPL) associée au premier voxel (V1) a lieu à titre de correction.

9. Dispositif pour déterminer la portée d'un rayonnement, en particulier un rayonnement particulaire, avec une unité de calcul (4) qui est configurée pour réaliser le procédé selon la revendication 1 moyennant une technique de programmation.
